# EUROPEAN PATENT APPLICATION

(11) **EP 4 810 055 A1**
(43) Date of publication of application: **23.09.2026**
(21) Application number: 24891681.9
(22) Date of filing: 30.10.2024
(51) Int. Cl.: A61K 38/08, A61P 25/02, A23L 33/18

(54) **PEPTIDE FOR TREATING DEMYELINATING DISEASES AND USE THEREOF**

(30) Priority: 15.11.2023 KR 20230158384
(71) Applicant: Kine Sciences Co., Ltd., Seoul 06221 (KR)
(72) Inventor: KIM, Myun Soo, Seoul 06221 (KR); BYUN, Jae Hyuk, Seoul 06221 (KR); GU, Changkyu, Seoul 06221 (KR)
(74) Representative: Vossius & Partner Patentanwälte Rechtsanwälte mbB
(86) International application number: PCT/KR2024/016753
(87) International publication number: WO 2025/105734

(57) **Abstract**

The present invention relates to a peptide for treating a demyelinating disease and a use thereof. More specifically, it was confirmed that the trimeric peptide according to the present invention suppresses demyelination and enhances myelination in a chronic inflammatory demyelinating polyneuropathy (CIDP) animal model and an acquired peripheral nerve injury animal model, and improves nerve damage and symptoms caused by demyelination. Accordingly, the peptide of the present invention can be usefully employed as an active ingredient in a composition for the prevention or treatment of demyelinating diseases, including CIDP.

## Description

### [Technical Field]

The present invention relates to a peptide for treating a demyelinating disease and a use thereof, and more particularly, to a use of a trimeric peptide consisting of the amino acid sequence set forth in SEQ ID NO: 1 as a therapeutic agent for treating a demyelinating disease.

### [Background Art]

Most nerve fibers in the nervous system are structurally surrounded by multiple layers of a tissue composed of a lipoprotein called myelin, and since myelin functions similarly to an electrical insulator, it plays an important role in enabling the accurate and rapid transmission of neural impulses along nerve fibers. When such myelin is damaged or destroyed due to localized injury, immune disorders, infection, nutritional deficiency, drugs, or unknown causes, permanent damage to the underlying nerve fibers is induced. Accordingly, when myelin is damaged in the body, remyelination proceeds in an attempt to restore the myelin; however, in many cases, remyelination fails and progresses to demyelination. Diseases caused by such demyelination are referred to as demyelinating diseases.

Demyelinating diseases are conditions in which myelin is damaged without causing significant injury to the nerve cells themselves or to the axons, and such diseases may occur in both the peripheral nervous system and the central nervous system. Symptoms of demyelinating diseases include sensory symptoms, motor symptoms, and autonomic nervous system symptoms, and the sensory symptoms and motor symptoms are further classified into positive symptoms and negative symptoms. Negative motor symptoms are caused by conduction block or loss of motor nerve axons and manifest as muscle weakness. In contrast, positive motor symptoms result from abnormal activity occurring in peripheral nerves and are manifested as fasciculation, myokymia, tremor, and muscle cramp. Positive sensory symptoms include hypersensitivity, including pain, and dysesthesia, while negative sensory symptoms may include reduced sensation and numbness.

To date, drugs used for demyelinating diseases mainly aim to correct the underlying causative diseases or to control neuropathic pain, which is a symptom of demyelinating diseases, and there are very few fundamental therapeutic agents. For example, treatments used for chronic inflammatory demyelinating polyneuropathy (CIDP), which is one type of demyelinating disease, include steroid preparations (primarily prednisone), intravenous immunoglobulin (IVIg), and plasma exchange; however, some patients with CIDP do not show an adequate response to such treatments and therefore require attempts with other types of therapies, such as immunosuppressive agents. However, in the case of immunosuppressive agents, periodic evaluation of risks and benefits is required during use.

Accordingly, the present inventors, as a result of efforts to develop a therapeutic agent having effective therapeutic efficacy while minimizing side effects as a treatment, prepared a trimeric peptide composed of peptides of very small size, thereby minimizing side effects associated with the administration of exogenous substances. In addition, the present inventors confirmed, in a CIDP animal model and an acquired peripheral nerve injury animal model, that the peptide suppresses demyelination and enhances myelination, and improves nerve damage and symptoms caused by demyelination. Based on these findings, the present inventors completed the present invention by demonstrating that the peptide can be usefully employed as an active ingredient in a composition for the prevention or treatment of demyelinating diseases, including CIDP.

### [Prior Art Documents]

### [Patent Document]

Korean Patent Publication No. 10-2020-0138904

### [Non-Patent Document]

G. G. A. van Lieverloo et al., Corticosteroids in chronic inflammatory demyelinating polyneuropathy, J Neurol. 2018; 265(9): 2052-2059.

Vanden Bergh et al., European Academy of Neurology/Peripheral Nerve Society guideline on diagnosis and treatment of chronic inflammatory demyelinating polyneuropathy: Report of a joint Task Force-Second revision, Eur J Neurol. 2021 Nov;28(11):3556-3583.

### [Detailed Description of the Invention]

### [Technical Problem]

An object of the present invention is to provide a composition for the prevention, treatment, or amelioration of a demyelinating disease, comprising, as an active ingredient, a peptide consisting of the amino acid sequence set forth in SEQ ID NO: 1 or a polynucleotide encoding the same.

### [Technical Solution]

In order to achieve the object of the present invention, the present invention provides: a pharmaceutical composition for the prevention or treatment of a demyelinating disease, comprising, as an active ingredient, a peptide consisting of the amino acid sequence set forth in SEQ ID NO: 1 or a polynucleotide encoding the same; use of the peptide or the polynucleotide encoding the same for use in a pharmaceutical composition for the prevention or treatment of a demyelinating disease; use of the peptide or the polynucleotide encoding the same for preparing a pharmaceutical composition for the prevention or treatment of a demyelinating disease; and a method for preventing or treating a demyelinating disease, comprising administering to a subject a therapeutically effective amount of the peptide or the polynucleotide encoding the same.

The present invention also provides: a health functional food composition for the prevention or amelioration of a demyelinating disease, comprising, as an active ingredient, a peptide consisting of the amino acid sequence set forth in SEQ ID NO: 1 or a polynucleotide encoding the same; use of the peptide or the polynucleotide encoding the same for use in a health functional food composition for the prevention or amelioration of a demyelinating disease; use of the peptide or the polynucleotide encoding the same for preparing a health functional food composition for the prevention or amelioration of a demyelinating disease; and a method for ameliorating a demyelinating disease, comprising administering to a subject the peptide or the polynucleotide encoding the same.

### [Advantageous Effects of the Invention]

In the present invention, it was confirmed that the trimeric peptide according to the present invention suppresses demyelination and enhances myelination in a chronic inflammatory demyelinating polyneuropathy (CIDP) animal model and an acquired peripheral nerve injury animal model, and improves nerve damage and symptoms caused by demyelination. Therefore, the peptide of the present invention can be usefully employed as an active ingredient in a composition for the prevention or treatment of demyelinating diseases, including CIDP.

In addition, the trimeric peptide according to the present invention is composed of peptides having a very small size, thereby minimizing side effects associated with the administration of exogenous substances.

### [Brief Description of the Drawings]

Fig. 1 shows changes in clinical scores following administration of the trimeric peptide KINE-101 according to the present invention in a chronic inflammatory demyelinating polyneuropathy (CIDP) animal model.
Fig. 2 shows the degree of demyelination after administration of the trimeric peptide KINE-101 according to the present invention in a CIDP animal model.
Fig. 3 shows changes in clinical scores following administration of the trimeric peptide KINE-101 according to the present invention or intravenous immunoglobulin (IVIg) in a CIDP animal model (*: p<0.05, ***: p<0.001, ****: p<0.0001).
Fig. 4 shows changes in body weight following administration of the trimeric peptide KINE-101 according to the present invention or IVIg in a CIDP animal model (*: p<0.05, **: p<0.01, ***: p<0.001, ****: p<0.0001).
Fig. 5 shows changes in motor nerve conduction velocity (MNCV) and compound muscle action potential (CMAP) following administration of the trimeric peptide KINE-101 according to the present invention or IVIg in a CIDP animal model (*: p<0.05, **: p<0.01, ***: p<0.001, ****: p<0.0001).
Fig. 6 shows changes in sensory nerve conduction velocity (SNCV) and sensory nerve action potential (SNAP) following administration of the trimeric peptide KINE-101 according to the present invention or IVIg in a CIDP animal model (*: p<0.05, **: p<0.01, ***: p<0.001).
Fig. 7 shows changes in MNCV following administration of the trimeric peptide KINE-101 according to the present invention in an acquired peripheral nerve injury animal model.
Fig. 8 shows changes in CMAP following administration of the trimeric peptide KINE-101 according to the present invention in an acquired peripheral nerve injury animal model.
Fig. 9 shows changes in SNCV following administration of the trimeric peptide KINE-101 according to the present invention in an acquired peripheral nerve injury animal model.
Fig. 10 shows changes in SNAP following administration of the trimeric peptide KINE-101 according to the present invention in an acquired peripheral nerve injury animal model.
Fig. 11 shows behavioral changes following administration of the trimeric peptide KINE-101 according to the present invention in an acquired peripheral nerve injury animal model.
Fig. 12a and Fig. 12b show the degree of myelin abnormality after administration of the trimeric peptide KINE-101 according to the present invention in an acquired peripheral nerve injury animal model.
Fig. 13 shows changes in the proportion and activity of Treg cells after administration of the trimeric peptide KINE-101 according to the present invention in an acquired peripheral nerve injury animal model.

### [Best Mode for Carrying Out the Invention]

Hereinafter, the present invention will be described in more detail.

The present invention provides a peptide consisting of the amino acid sequence set forth in SEQ ID NO: 1 or a polynucleotide encoding the same.

As used herein, the term "peptide" refers to a polymer composed of two or more amino acids linked by an amide bond (or peptide bond). Despite various studies on peptide therapeutics, the size of peptides themselves is often too large, resulting in the generation of antibodies against the peptide drugs and the induction of immune responses, thereby causing reduced efficacy of the drugs and adverse side effects. In this regard, the present invention is technically significant in that it identifies a trimeric peptide having pharmaceutically effective activity and consisting of 10 or fewer amino acids.

The peptide of the present invention may consist of the amino acid sequence set forth in SEQ ID NO: 1, and may include an amino acid sequence having at least 75% sequence homology, preferably at least 80% sequence homology, more preferably at least 90% sequence homology, and most preferably at least 95% sequence homology with the amino acid sequence set forth in SEQ ID NO: 1. More specifically, the peptide of the present invention may include an amino acid sequence having 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.5% or more sequence homology with the amino acid sequence set forth in SEQ ID NO: 1.

In addition, the peptide of the present invention may further comprise an amino acid sequence prepared for a specific purpose, such as a targeting sequence, a tag, a labeled residue, or a sequence for increasing the half-life or stability of the peptide.

In addition, the peptide of the present invention may be obtained by various methods widely known in the art. For example, the peptide may be produced by a method of preparing it using polynucleotide recombination and a protein expression system, by synthesizing it in vitro through chemical synthesis such as peptide synthesis, or by a cell-free protein synthesis method.

In addition, in order to obtain improved chemical stability, enhanced pharmacological properties (such as half-life, absorbability, potency, and efficacy), altered specificity (for example, a broader spectrum of biological activity), and reduced antigenicity, a protecting group may be bound to the N- or C-terminus of the peptide. Examples of such protecting groups include an acetyl group, a fluorenyl methoxy carbonyl group, a formyl group, a palmitoyl group, a myristyl group, a stearyl group, or polyethylene glycol (PEG). However, the peptide is not limited thereto and may include, without limitation, any component capable of modifying the peptide, particularly improving the stability of the peptide.

As used herein, the term "stability" refers not only to in vivo stability that protects the peptide of the present invention from attack by proteolytic enzymes in the body, but also to storage stability (e.g., stability during storage at room temperature).

As used herein, the term "polynucleotide" refers to a polymer in which nucleotides are linked together and which functions to transmit genetic information. For the purposes of the present invention, the polynucleotide encodes the peptide of SEQ ID NO: 1 and may include a sequence having at least 75% sequence homology, preferably at least 85% sequence homology, more preferably at least 90% sequence homology, and most preferably at least 95% sequence homology with a polynucleotide sequence encoding the peptide.

As used herein, the term "homology" refers to the degree of similarity with a wild-type amino acid sequence or a polynucleotide sequence. Such comparison of homology may be performed using comparison programs widely known in the art, and the homology between two or more sequences may be calculated as a percentage (%).

In addition, the present invention provides: a pharmaceutical composition for preventing or treating a demyelinating disease, comprising, as an active ingredient, a peptide consisting of the amino acid sequence set forth in SEQ ID NO: 1 or a polynucleotide encoding the same; use of the peptide or the polynucleotide encoding the same for use in a pharmaceutical composition for preventing or treating a demyelinating disease; use of the peptide or the polynucleotide encoding the same for preparing a pharmaceutical composition for preventing or treating a demyelinating disease; and a method for preventing or treating a demyelinating disease, comprising administering to a subject a therapeutically effective amount of the peptide or the polynucleotide encoding the same.

As used herein, the term "prevention" refers to any act of suppressing a disease or delaying the onset of the disease by administration of the pharmaceutical composition according to the present invention.

As used herein, the term "treatment" refers to any act in which the symptoms of a disease are improved or beneficially changed by administration of the pharmaceutical composition according to the present invention.

As used herein, the term "subject" refers to a target requiring treatment of a disease, and more specifically refers to a mammal such as a human or a non-human primate, a mouse, a dog, a cat, a horse, or a cow.

In the present invention, the demyelinating disease may be, but is not limited to, Charcot-Marie-Tooth disease (CMT), chronic inflammatory demyelinating polyneuropathy (CIDP), idiopathic inflammatory demyelinating disease, hereditary neuropathy, anti-MAG peripheral neuropathy, progressive inflammatory neuropathy, optic neuropathy, Devic's disease, central pontine myelinolysis (CPM), extrapontine myelinolysis (EPM), tabes dorsalis, leukoencephalopathies, demyelinating leukodystrophy, neuromyelitis optica, demyelinating optic neuritis, acute disseminated demyelination, periaxial encephalitis, central demyelination of the corpus callosum, acute transverse myelitis, subacute necrotizing myelitis, or concentric sclerosis.

In addition, the peptide may prevent or treat a demyelinating disease by improving demyelination.

In the present invention, the peptide may prevent or treat the demyelinating disease by improving damage to motor nerves and sensory nerves, specifically damage to motor nerves and sensory nerves caused by demyelination.

In addition, the peptide may prevent or treat a demyelinating disease by improving decreases in motor nerve conduction and sensory nerve conduction, specifically decreases in motor nerve conduction and sensory nerve conduction caused by demyelination.

In addition, the peptide may prevent or treat a demyelinating disease by increasing the proportion of Treg cells in lymph nodes at a peripheral nerve injury site and inducing activation of the Treg cells.

In specific embodiments of the present invention, the present inventors prepared a trimeric peptide, KINE-101, using a PSP fragment (PSP monomer), and confirmed, in a CIDP animal model as a demyelinating disease model, that the peptide improved CIDP symptoms and ameliorated demyelination in peripheral nerve tissues. In addition, it was confirmed that decreases in motor nerve conduction and sensory nerve conduction caused by demyelination were improved.

In addition, the present inventors confirmed, in an acquired peripheral nerve injury animal model as a demyelinating disease model, that the peptide improved symptoms caused by motor nerve and sensory nerve damage, such as decreased muscle strength and sensory impairment, and ameliorated demyelination, which is a form of peripheral nerve injury.

Accordingly, the present inventors confirmed that the peptide of the present invention suppresses demyelination and enhances myelination in a CIDP animal model and an acquired peripheral nerve injury animal model, and improves nerve damage and symptoms caused by demyelination. Therefore, the peptide of the present invention can be usefully employed as an active ingredient in a pharmaceutical composition for preventing or treating demyelinating diseases, including CIDP.

The peptide of the present invention or the polynucleotide encoding the same may be delivered in a pharmaceutically acceptable carrier such as a colloidal suspension, powder, saline, lipid, liposome, microspheres, or nanospheres. These may form a complex with or be associated with a delivery vehicle and may be delivered in vivo using delivery systems known in the art, such as lipids, liposomes, microparticles, gold, nanoparticles, polymers, condensation reagents, polysaccharides, polyamino acids, dendrimers, saponins, absorption-enhancing agents, or fatty acids.

In addition, pharmaceutically acceptable carriers may include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, which are commonly used in formulation. In addition to the above components, lubricants, wetting agents, sweetening agents, flavoring agents, emulsifying agents, suspending agents, preservatives, and the like may be further included.

The pharmaceutical composition of the present invention may be administered orally or parenterally (for example, intramuscularly, intravenously, intraperitoneally, subcutaneously, intradermally, or topically) depending on the intended method, and the dosage may vary depending on the condition and body weight of the patient, the severity of the disease, the drug formulation, the route of administration, and the time of administration, but may be appropriately selected by a person skilled in the art.

The pharmaceutical composition of the present invention is administered in a pharmaceutically effective amount. As used herein, the term "pharmaceutically effective amount" refers to an amount sufficient to treat a disease at a reasonable benefit/risk ratio applicable to medical treatment. The effective dosage level may be determined depending on factors including the type and severity of the patient's disease, the activity of the drug, sensitivity to the drug, the time of administration, the route of administration and rate of excretion, the duration of treatment, drugs used in combination, and other factors well known in the medical field. The pharmaceutical composition according to the present invention may be administered as a single therapeutic agent or in combination with other therapeutic agents, and may be administered simultaneously with, separately from, or sequentially with conventional therapeutic agents, and may be administered in a single dose or multiple doses. Considering all of the above factors, it is important to administer an amount that can obtain the maximum effect with the minimum amount without causing side effects, and such an amount may be readily determined by those skilled in the art.

Specifically, the effective amount of the pharmaceutical composition of the present invention may vary depending on the patient's age, sex, condition, body weight, the absorption of the active ingredient in the body, the rate of inactivation, the rate of excretion, the type of disease, and drugs administered in combination, and may be increased or decreased depending on the route of administration, the severity of the condition, sex, body weight, and age.

In addition, the present invention provides: a health functional food composition for preventing or ameliorating a demyelinating disease, comprising, as an active ingredient, a peptide consisting of the amino acid sequence set forth in SEQ ID NO: 1 or a polynucleotide encoding the same; use of the peptide or the polynucleotide encoding the same for use in a health functional food composition for preventing or ameliorating a demyelinating disease; use of the peptide or the polynucleotide encoding the same for preparing a health functional food composition for preventing or ameliorating a demyelinating disease; and a method for ameliorating a demyelinating disease, comprising administering to a subject the peptide or the polynucleotide encoding the same.

As used herein, the term "amelioration" refers to any act that at least reduces parameters associated with the condition being treated, for example, the severity of symptoms.

In the present invention, the descriptions of the peptide, the polynucleotide, and the demyelinating disease are the same as described above; therefore, detailed descriptions thereof will be incorporated by reference to the above description, and hereinafter only the specific components of the health functional food composition will be described.

Meanwhile, the present inventors confirmed that the peptide of the present invention suppresses demyelination and enhances myelination in a CIDP animal model and an acquired peripheral nerve injury animal model, and improves nerve damage and symptoms caused by demyelination. Accordingly, the peptide of the present invention can be usefully employed as an active ingredient in a health functional food composition for preventing or ameliorating demyelinating diseases, including CIDP.

The health functional food composition of the present invention may be used for the prevention or amelioration of a disease before the onset of the disease or after the onset thereof, and may be used simultaneously with or separately from a therapeutic agent for treatment.

In the health functional food composition of the present invention, the active ingredient may be added to food as it is or used together with other foods or food ingredients, and may be appropriately used according to conventional methods. The amount of the active ingredient mixed may be suitably determined depending on its intended use (for prevention or amelioration). In general, when manufacturing food or beverages, the composition of the present invention may be added in an amount of preferably 15% by weight or less, and more preferably 10% by weight or less, based on the raw materials. However, in the case of long-term intake for the purpose of health and hygiene or for health control, the above amount may be less than the above range.

The health functional food composition of the present invention may contain, in addition to the active ingredient, other components as essential ingredients without particular limitation. For example, various flavoring agents or natural carbohydrates may be included as additional components, as in conventional beverages. Examples of the above-described natural carbohydrates include monosaccharides such as glucose and fructose; disaccharides such as maltose and sucrose; and polysaccharides such as dextrin and cyclodextrin, which are conventional sugars, as well as sugar alcohols such as xylitol, sorbitol, and erythritol. As flavoring agents other than those described above, natural flavoring agents (e.g., thaumatin, stevia extract (for example, rebaudioside A, glycyrrhizin, etc.)) and synthetic flavoring agents (e.g., saccharin, aspartame, etc.) may be advantageously used. The proportion of the natural carbohydrates may be appropriately determined by those skilled in the art.

In addition, the health functional food composition of the present invention may further contain various nutrients, vitamins, minerals (electrolytes), flavoring agents such as synthetic flavoring agents and natural flavoring agents, coloring agents, and fillers (e.g., cheese, chocolate), pectic acid and salts thereof, alginic acid and salts thereof, organic acids, protective colloid thickeners, pH adjusting agents, stabilizers, preservatives, glycerin, alcohol, and carbonating agents used in carbonated beverages. These components may be used independently or in combination, and the proportions of such additives may also be appropriately selected by those skilled in the art.

Hereinafter, the present invention will be described in detail with reference to the following Examples.

However, the following Examples are provided for illustrative purposes only, and the scope of the present invention is not limited thereto.

### <Example 1> Preparation of Peptide

In this Example, a trimeric polymer, KINE-101, was prepared using a PSP fragment (PSP monomer), as shown in Table 1 below. Thereafter, the synthesized peptide was purified by high-performance liquid chromatography (SHIMADZU Prominence HPLC), and a Shiseido Capcell Pak C18 column (4.6 × 50 mm) was used. In addition, the mass of the synthesized peptide was confirmed using a mass spectrometer (AXIMA Assurance, MALDI-TOF, Shimadzu).

**[Table 1]**

| Peptide Name | Amino Acid Sequence | Molecular Weight (MW) |
|---|---|---|
| KINE-101 | PSPPSPPSP (SEQ ID NO: 1) | 861.9 g/mol |

### <Example 2> Evaluation of the Effect of KINE-101 on the Improvement of Chronic Inflammatory Demyelinating Polyneuropathy (CIDP) in a CIDP Animal Model

### <2-1> Preparation of CIDP Animal Model and Peptide Administration

In order to evaluate the effect of KINE-101 on demyelinating diseases, a chronic inflammatory demyelinating polyneuropathy (CIDP) animal model, which is a form of demyelinating disease, was prepared as follows, and KINE-101 synthesized in <Example 1> was administered.

Female Lewis rats (7 weeks old) were obtained and acclimatized for one week. Thereafter, solution A was prepared by dissolving peripheral myelin P0 protein (180-199) in 0.9% saline at a concentration of 300 µg/µl, and solution B was prepared by dissolving *Mycobacterium tuberculosis* in Freund's incomplete adjuvant at a concentration of 0.5 mg/µl. A sterile 50 ml tube was placed in an ice box, and solutions A and B were mixed at a ratio of 1:1. The mixture was then homogenized using a homogenizer for 15 minutes to prepare an emulsion. The prepared emulsion was loaded into a 1 ml syringe, the syringe needle was replaced with a 25G needle, and the syringe was stored in an ice box containing ice until use. The emulsion (200 µL) was subcutaneously (S.C.) injected into the tail of the Lewis rats to induce the disease. Since body weight decreases depending on the severity of CIDP, the animals were divided into six groups as shown in Table 2 below, and the groups were separated after adjusting the average body weight of the induced groups as closely as possible. From the day following disease induction, KINE-101 dissolved in DPBS was intravenously (I.V.) administered three times per week at doses of 1, 5, 25, and 50 mg/kg.

**[Table 2]**

| Group | Administered Substance (Route of Administration) |
|---|---|
| Normal | - |
| EAN P0(180-199) | Emulsion 200 µL (S.C.) + DPBS |
| KINE-101(1mg/kg) | Emulsion 200 µL (S.C.) + KINE-101 1 mg/kg (I.V.) |
| KINE-101(5mg/kg) | Emulsion 200 µL (S.C.) + KINE-101 5 mg/kg (I.V.) |
| KINE-101(25mg/kg) | Emulsion 200 µL (S.C.) + KINE-101 25 mg/kg (I.V.) |
| KINE-101(50mg/kg) | Emulsion 200 µL (S.C.) + KINE-101 50 mg/kg (I.V.) |

### <2-2> Evaluation of the Symptom-Improving Effect of the Peptide in a CIDP Animal Model

In order to examine the progression of CIDP following administration of KINE-101, the severity of CIDP over time after administration of KINE-101 to the CIDP animal model was evaluated and measured using a clinical score.

Specifically, during administration of KINE-101 by the method described in Example <2-1>, the symptoms were evaluated on a scale of 0 to 5 according to the criteria shown in Table 3 below. The clinical scoring system was based on Brain Behav Immun. 2007 Jul;21(5):699-710. The severity of CIDP was quantified by calculating the average value of the results evaluated for each individual.

**[Table 3]**

| Score | Symptom |
|---|---|
| 0 | No limping of the legs, and no curling of the tail when the observer's hand is brought close to the tail |
| 1 | When the sole of the foot is turned upward, the hind paw remains facing upward for more than 3 seconds |
| 2 | Loss of strength in both the hind legs and the tail |
| 3 | Paralysis in one of the two hind legs, causing it to remain facing upward at all times |
| 4 | Paralysis in both hind legs, causing them to remain facing upward at all times |
| 5 | Moribund state or death |

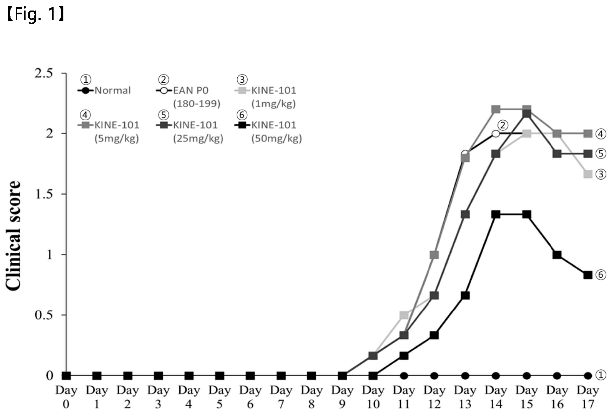

As a result, as shown in Fig. 1, no change in clinical score was observed in all groups except the normal group (Normal) until day 10, at which time symptoms of loss of tail strength began to appear. Meanwhile, the CIDP-induced group (EAN P0 (180-199)) showed persistent symptoms after day 14, whereas all KINE-101-treated groups exhibited alleviation of symptoms after day 15. In particular, the KINE-101 50 mg/kg treatment group showed the lowest clinical score. These results indicate that the peptide of the present invention alleviates CIDP symptoms.

### <2-3> Evaluation of the Demyelination-Improving Effect of the Peptide in a CIDP Animal Model

After administration of KINE-101 to the CIDP animal model, demyelination in peripheral nerve tissues was observed.

Specifically, after completion of the experiment, the experimental animals in the KINE-101 (50 mg/kg) group, which showed the most significant effect in alleviating CIDP symptoms in Example <2-2>, were sacrificed, and peripheral nerve tissues of the hind limbs were isolated. Tissue cross-sections were then observed using a transmission electron microscope (TEM) to evaluate the degree of myelin loss. In addition, comparisons were made with the normal group (Normal) and the CIDP-induced group (EAN P0 (180-199)).

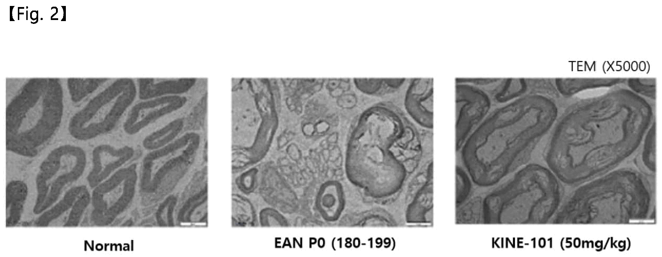

As a result, as shown in Fig. 2, demyelination and resulting structural changes were observed in the nerves of the CIDP-induced group compared with the normal group, whereas in the KINE-101 (50 mg/kg) group, demyelination was suppressed similarly to that observed in the normal group.

### <Example 3> Comparison of the Effects of KINE-101 and Intravenous Immunoglobulin (IVIg) in a CIDP Animal Model

### <3-1> Preparation of CIDP Animal Model and Administration of Peptide or Intravenous Immunoglobulin (IVIg)

In order to evaluate the effect of KINE-101 compared with existing therapeutic methods, a CIDP animal model in which the disease persists for a long period was established, and KINE-101 synthesized in <Example 1> or intravenous immunoglobulin (IVIg), which is used as a therapeutic agent for CIDP, was administered.

Specifically, male Lewis rats (7 weeks old) were obtained and acclimatized for one week. Thereafter, solution A was prepared by dissolving s-palm P0, in which palmitic acid is conjugated to cysteine at the 181st amino acid of peripheral myelin P0 protein described in <Example 2>, in saline at a concentration of 2 mg/mL. Solution B was prepared by dissolving *Mycobacterium tuberculosis* in Freund's adjuvant at a concentration of 5 mg/mL. A sterile 25 mL tube was placed in an ice box, and solutions A and B were mixed at a ratio of 1:1 and homogenized for 15 minutes using a homogenizer to prepare an emulsion. The emulsion (200 µL) was then administered by subcutaneous injection at the base of the rat tail to induce the disease. Since body weight decreases depending on the severity of CIDP, the animals were divided into seven groups as shown in Table 4 below, with the groups separated after adjusting the average body weight of the induced groups as closely as possible. The KINE-101 administration groups received KINE-101 dissolved in DPBS at doses of 6.25, 12.5, 25, and 37.5 mg/kg by intravenous injection (I.V.) three times per week for 15 days, starting the day after disease induction. The IVIg administration group received IVIg at a dose of 400 mg/kg once daily for 5 days starting the day after disease induction. The experiment was terminated on day 15 after disease induction.

**[Table 4]**

| Group | Administered Substance (Route of Administration) |
|---|---|
| Control | - |
| S-palmP0(180-199) + saline | Emulsion 200 µL (S.C.) + DPBS |
| S-palmP0(180-199) + KINE-101 6.25mg/kg | Emulsion 200 µL (S.C.) + KINE-101 6.25 mg/kg (I.V.) |
| S-palmP0(180-199) + KINE-101 12.5mg/kg | Emulsion 200 µL (S.C.) + KINE-101 12.5 mg/kg (I.V.) |
| S-palmP0(180-199) + KINE-101 25mg/kg | Emulsion 200 µL (S.C.) + KINE-101 25 mg/kg (I.V.) |
| S-palmP0(180-199) + KINE-101 37.5mg/kg | Emulsion 200 µL (S.C.) + KINE-101 37.5 mg/kg (I.V.) |
| S-palmP0(180-199) + | Emulsion 200 µL (S.C.) + |
| IVIg 400mg/kg | IVIg 400mg/kg (I.V.) |

### <3-2> Comparison of the Symptom-Improving Effects of the Peptide and IVIg in a CIDP Animal Model

In order to evaluate the effect of KINE-101 compared with existing therapeutic methods, the severity of CIDP over time after administration of KINE-101 or IVIg to the CIDP animal model was evaluated and measured using a clinical score.

Specifically, during administration of KINE-101 or IVIg by the method described in Example <3-1>, the induction and progression of the disease were observed by monitoring the movement of the tail and hind limbs and were evaluated on a scale of 0 to 5 according to the symptoms as shown in Table 5 below (PMID: 25595246). The severity of CIDP was quantified by calculating the average value of the results evaluated for each individual.

**[Table 5]**

| Score | Symptom |
|---|---|
| 0 | Normal |
| 1 | Loss of tail strength |
| 2 | Gait abnormality |
| 3 | Paralysis of one hind limb |
| 4 | Paralysis of both hind limbs |
| 5 | Death |

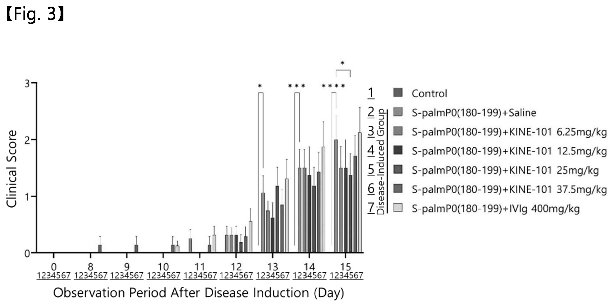

As a result, as shown in Fig. 3, the negative control group (S-palmP0 (180-199) + saline) began to exhibit loss of tail strength on day 11 after disease induction, and gait abnormalities were observed on day 15, which was the final day of the experiment. This indicates that the disease was successfully induced. In contrast, all KINE-101-treated groups (6.25 mg/kg to 37.5 mg/kg) showed lower clinical scores on day 15 after disease induction compared with the negative control group, whereas the IVIg-treated group, used as a comparison group, showed clinical scores similar to or higher than those of the negative control group. In particular, the KINE-101 25 mg/kg treatment group showed the lowest clinical score. These results indicate that the peptide of the present invention exhibits superior CIDP symptom-alleviating effects compared with IVIg for the treatment of CIDP.

### <3-3> Comparison of the Effects of the Peptide and IVIg on Body Weight in a CIDP Animal Model

Demyelinating diseases such as CIDP are known to cause reduced body weight gain due to limited movement resulting from contraction and paralysis of peripheral muscles, which leads to insufficient food intake. Accordingly, in order to evaluate the effect of KINE-101 compared with existing therapeutic methods, changes in body weight over time were measured after administration of KINE-101 or IVIg in the CIDP animal model.

Specifically, body weight was measured during administration of KINE-101 or IVIg by the method described in Example <3-1>, and the change in body weight was calculated relative to the body weight before disease induction (Day 0).

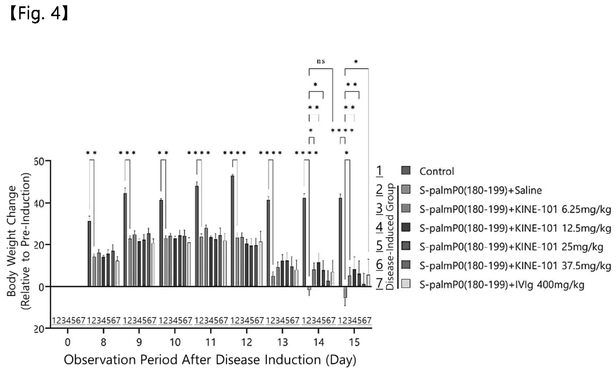

As a result, as shown in Fig. 4, the negative control group (S-palmP0 (180-199) + saline) exhibited approximately a 50% reduction in body weight gain on day 8 after disease induction compared with the normal group (Control), and on day 15 after disease induction, a decrease in body weight compared with that before disease induction (Day 0) was observed. In contrast, the KINE-101-treated groups showed greater body weight gain compared with the negative control group and maintained significantly higher body weight than that before disease induction even on day 15 after disease induction. In addition, except for days 14 and 15 after disease induction, all KINE-101-treated groups maintained body weight gain similar to or higher than that of the IVIg-treated group. In particular, the KINE-101 12.5 mg/kg treatment group showed the most pronounced body weight improvement effect. These results indicate that the peptide of the present invention exhibits body weight improvement effects similar to or superior to those of IVIg.

### <3-4> Comparison of the Nerve Conduction-Improving Effects of the Peptide and IVIg in a CIDP Animal Model

Demyelination is known to cause a decrease in nerve conduction velocity and conduction block. Accordingly, in order to evaluate the effect of KINE-101 compared with existing therapeutic methods, nerve conduction studies (NCS) were performed by electrophysiological examination after administration of KINE-101 or IVIg in the CIDP animal model.

Specifically, KINE-101 or IVIg was administered by the method described in Example <3-1>, and after completion of the experiment, the animals were anesthetized with 1.5% isoflurane and examined. The fur from the distal region of the hind limb to the upper region was removed, and nerve conduction studies for measuring motor and sensory nerves were performed using a Nicolet VikingQuest device as the electrophysiological examination instrument. For the nerve conduction study, a needle electrode was placed on the motor nerve or sensory nerve, and a reference electrode was placed on the gastrocnemius muscle. The stimulating cathode was positioned in the middle of the posterior thigh near the recording electrode, and stimulation was applied at both distal and proximal sites. Motor nerve conduction velocity (MNCV) and compound muscle action potential (CMAP) of the motor nerve, as well as sensory nerve conduction velocity (SNCV) and sensory nerve action potential (SNAP) of the sensory nerve, were measured.

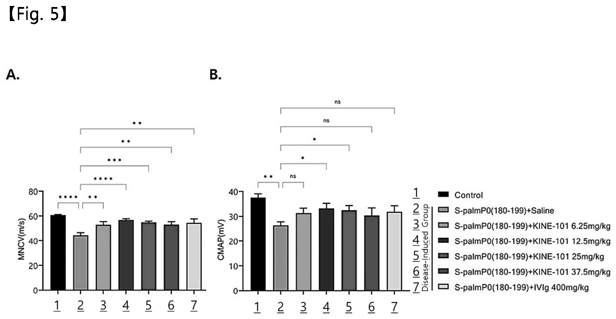

As a result, as shown in Fig. 5, the negative control group (S-palmP0 (180-199) + saline) showed decreased MNCV and CMAP compared with the normal group (Control). In contrast, all KINE-101-treated groups showed higher MNCV and CMAP values compared with the negative control group and exhibited effects similar to or superior to those of the IVIg-treated group. In particular, the KINE-101 12.5 mg/kg treatment group showed the most pronounced effect.

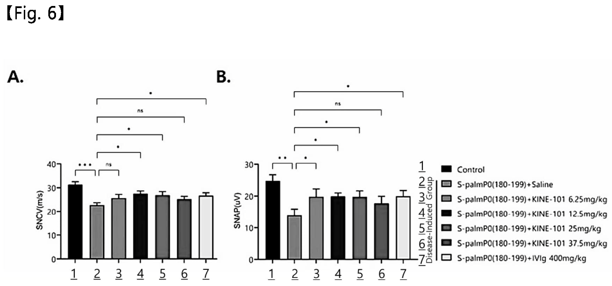

In addition, as shown in Fig. 6, the negative control group (S-palmP0 (180-199) + saline) exhibited decreased SNCV and SNAP compared with the normal group (Control). In contrast, all KINE-101-treated groups showed higher SNCV and SNAP values compared with the negative control group and exhibited effects similar to those of the IVIg-treated group. In particular, the KINE-101 12.5 mg/kg treatment group showed the most pronounced effect.

From the above results, it can be confirmed that the peptide of the present invention exhibits nerve conduction-improving effects that are superior to IVIg (motor nerve conduction) or comparable to IVIg (sensory nerve conduction).

In addition, considering the results of Examples <3-2> to <3-4>, it can be confirmed that the peptide of the present invention exhibits superior therapeutic effects compared with IVIg for the treatment of CIDP.

### <Example 4> Evaluation of the Nerve Damage-Improving Effect of KINE-101 in an Acquired Peripheral Nerve Injury Animal Model

### <4-1> Preparation of Acquired Peripheral Nerve Injury Animal Model and Peptide Administration

Demyelinating diseases occur when the myelin sheath constituting nerves is damaged and may occur in both the peripheral nervous system and the central nervous system. Accordingly, in order to evaluate the effect of KINE-101 on demyelinating diseases, an acquired peripheral nerve injury animal model was prepared as follows, and KINE-101 synthesized in <Example 1> was administered.

Specifically, a uniform nerve injury model can be obtained by damaging the nerve emerging from the spinal cord at a predetermined intensity for a certain period of time, and since both the intensity and duration can be controlled, it is possible to establish a nerve injury model that persists for a desired period. Accordingly, C57BL/6 mice were obtained and acclimatized for one week and then divided into five groups as shown in Table 6 below. For the disease induction groups, the boundary region between the gluteal muscle and the femoral muscle of the mouse was incised to expose the sciatic nerve. The nerve was then grasped and injured for a predetermined period using a surgical tool (MacJam) at one of three intensities (stages 1, 2, or 3; stage 3 for 3 seconds was used as the reference), after which the incision was sutured to establish an acquired peripheral nerve injury animal model. From the day following disease induction, KINE-101 dissolved in DPBS was administered intravenously (I.V.) at doses of 10, 50, and 100 mg/kg three times per week for a total of four weeks.

**[Table 6]**

| Group | Administered Substance (Route of Administration) |
|---|---|
| WT Ctr | - |
| Injury Ctr | Nerve Injury + DPBS |
| Injury KINE-101(10mg/kg) | Nerve Injury + KINE-101 10 mg/kg (I.V.) |
| Injury KINE-101(50mg/kg) | Nerve Injury + KINE-101 50 mg/kg (I.V.) |
| Injury KINE-101(100mg/kg) | Nerve Injury + KINE-101 100 mg/kg (I.V.) |

### <4-2> Evaluation of the Nerve Damage-Improving Effect of the Peptide in an Acquired Peripheral Nerve Injury Animal Model

In order to evaluate the effect of KINE-101 on demyelinating diseases, nerve conduction studies were performed by electrophysiological examination after administration of KINE-101 in the acquired peripheral nerve injury animal model.

Specifically, mice were anesthetized with 1.5% isoflurane at weeks 0, 3, and 6 after administration of KINE-101 according to the method described in Example <4-1>, and MNCV, CMAP, SNCV, and SNAP were measured by the same method described in Example <3-4> to determine whether motor nerves and sensory nerves were damaged.

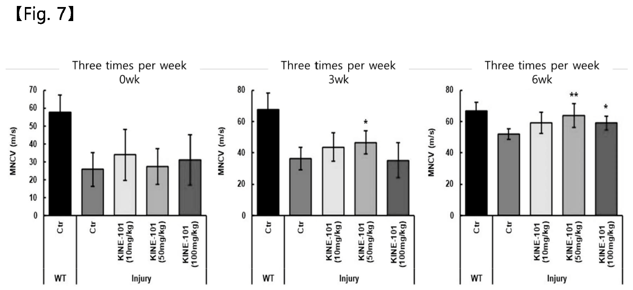

As a result, as shown in Fig. 7, MNCV in the acquired peripheral nerve injury group (Injury Ctr) was measured at 25.8 ± 9.4 m/s, confirming electrophysiologically that motor nerves were damaged compared with the normal group (WT Ctr, 57.9 ± 9.5 m/s). In contrast, at week 3 after administration of KINE-101 three times per week for four weeks following induction of acquired peripheral nerve injury, the MNCV of the KINE-101 50 mg/kg treatment group was 46.7 ± 7.4 m/s (p = 0.030), which was statistically significantly increased compared with the acquired peripheral nerve injury group (36.3 ± 7.2 m/s). In addition, at week 6 after administration of KINE-101 three times per week for four weeks, the MNCV of the KINE-101-treated groups increased, and in particular, the MNCV of the KINE-101 50 mg/kg treatment group (63.9 ± 7.6 m/s, p = 0.008) and the KINE-101 100 mg/kg treatment group (59.0 ± 4.5 m/s, p = 0.013) was statistically significantly increased compared with that of the acquired peripheral nerve injury group.

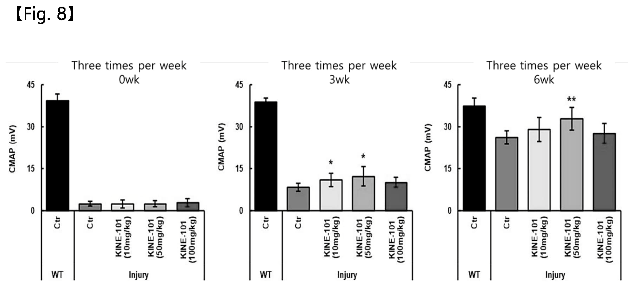

In addition, as shown in Fig. 8, CMAP in the acquired peripheral nerve injury group was measured at 2.5 ± 0.87 mV, confirming electrophysiologically that motor nerves were damaged compared with the normal group (39.3 ± 2.4 mV). In contrast, at weeks 3 and 6 after administration of KINE-101 three times per week for four weeks following induction of acquired peripheral nerve injury, CMAP values in the KINE-101-treated groups increased compared with those in the acquired peripheral nerve injury group. In particular, at week 3 after administration of KINE-101 three times per week for four weeks following induction of acquired peripheral nerve injury, statistically significant increases were observed in the KINE-101 10 mg/kg treatment group (11.0 ± 2.3 mV, p = 0.044) and the KINE-101 50 mg/kg treatment group (12.3 ± 3.5 mV, p = 0.039) compared with the acquired peripheral nerve injury group. Furthermore, at week 6 after administration of KINE-101 three times per week for four weeks following induction of acquired peripheral nerve injury, a statistically significant increase was observed in the KINE-101 50 mg/kg treatment group (32.9 ± 4.0 mV, p = 0.007) compared with the acquired peripheral nerve injury group.

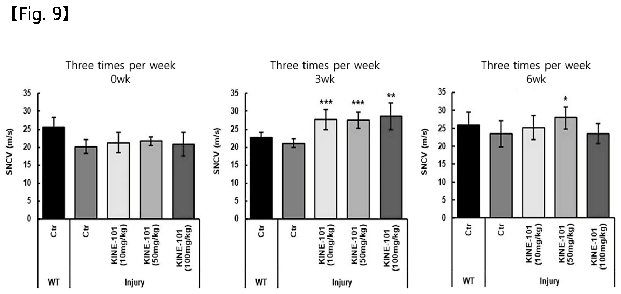

In addition, as shown in Fig. 9, SNCV in the acquired peripheral nerve injury group was measured at 20.2 ± 1.9 m/s, confirming electrophysiologically that sensory nerves were damaged compared with the normal group (25.6 ± 2.6 m/s). In contrast, at week 3 after administration of KINE-101 three times per week for four weeks following induction of acquired peripheral nerve injury, SNCV values in the KINE-101 10 mg/kg treatment group (27.7 ± 2.8 m/s, p = 0.000), the KINE-101 50 mg/kg treatment group (27.6 ± 2.2 m/s, p = 0.000), and the KINE-101 100 mg/kg treatment group (28.6 ± 3.7 m/s, p = 0.001) were all statistically significantly increased compared with the acquired peripheral nerve injury group (21.1 ± 1.2 m/s). At week 6 after administration of KINE-101 three times per week for four weeks following induction of acquired peripheral nerve injury, the SNCV of the KINE-101 50 mg/kg treatment group was 27.9 ± 3.1 m/s (p = 0.038), which was statistically significantly increased compared with that of the acquired peripheral nerve injury group (23.4 ± 3.6 m/s).

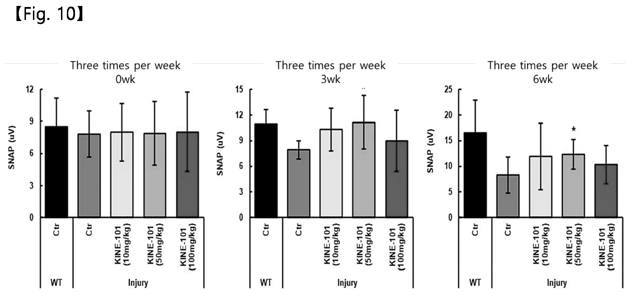

In addition, as shown in Fig. 10, SNAP in the acquired peripheral nerve injury group was measured at 7.8 ± 2.2 µV, confirming electrophysiologically that sensory nerves were damaged compared with the normal group (8.5 ± 2.6 µV). In contrast, at weeks 3 and 6 after administration of KINE-101 three times per week for four weeks following induction of acquired peripheral nerve injury, SNAP values in the KINE-101-treated groups increased compared with those in the acquired peripheral nerve injury group. In particular, at week 3 after administration of KINE-101 three times per week for four weeks following induction of acquired peripheral nerve injury, a statistically significant increase was observed in the KINE-101 50 mg/kg treatment group (11.2 ± 3.1 µV, p = 0.050) compared with the acquired peripheral nerve injury group (7.9 ± 1.1 µV). Furthermore, at week 6 after administration of KINE-101 three times per week for four weeks following induction of acquired peripheral nerve injury, a statistically significant increase was observed in the KINE-101 50 mg/kg treatment group (12.3 ± 2.9 µV, p = 0.046) compared with the acquired peripheral nerve injury group (8.3 ± 3.5 µV).

From the above results, it can be confirmed that the peptide of the present invention improves damage to the peripheral nervous system.

### <4-3> Evaluation of Symptom Improvement by the Peptide in an Acquired Peripheral Nerve Injury Animal Model

Demyelinating diseases are known to be accompanied by symptoms such as muscle weakness and impaired balance. Accordingly, a rotarod test was performed after administration of KINE-101 in the acquired peripheral nerve injury animal model.

Specifically, the phenotype of the experimental animals was analyzed using rotarod, grip strength, and tail suspension tests. The rotarod test was performed up to three times with a cut-off time of 3 minutes to evaluate muscle strength. Using a rotarod apparatus (ROTA ROD, LE8205, Panlab), the time during which the animal remained on the rotating rod was measured for 2 minutes and 30 seconds at a fixed speed of 8 rpm. For each evaluation, three trials were conducted, and the maximum duration among the trials was analyzed.

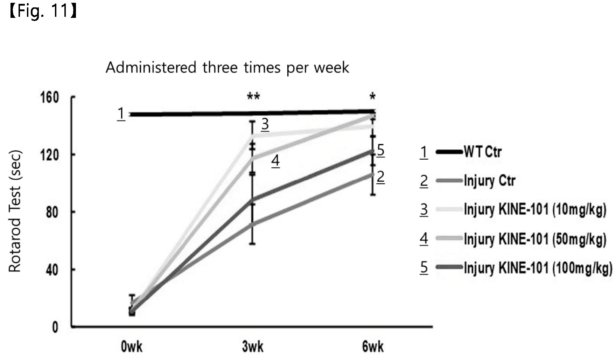

As a result, as shown in Fig. 11, at weeks 3 and 6 after administration of KINE-101 three times per week for four weeks following induction of acquired peripheral nerve injury, all KINE-101-treated groups exhibited improved muscle strength and balance compared with the acquired peripheral nerve injury group (Injury Ctr).

### <4-4> Evaluation of the Demyelination-Improving Effect of the Peptide in an Acquired Peripheral Nerve Injury Animal Model

After administration of KINE-101 to the acquired peripheral nerve injury animal model, histopathological examination of nerve tissues was performed.

Specifically, KINE-101 was administered to the acquired peripheral nerve injury animal model for four weeks according to the method described in Example <4-1>, and the experimental animals were sacrificed six weeks after the start of administration. Thereafter, peripheral nerve tissues of the hind limbs were isolated and fixed in a 4% paraformaldehyde solution containing 2.5% glutaraldehyde. After ethanol dehydration, the tissues were embedded in epoxy resin and semi-thin sections (1 µm) were prepared. The semi-thin sections were stained with toluidine blue for 5-10 seconds and observed under a light microscope. In addition, ultra-thin sections (65 nm) were prepared, and tissue cross-sections were observed using a transmission electron microscope (TEM) to examine abnormalities of myelin (demyelination, dysmyelination, and hypermyelination). Abnormally thickened and morphologically abnormal nerves were observed in the nerves of neuropathic mice, and abnormal myelin structures associated with axonal abnormalities were observed under the electron microscope.

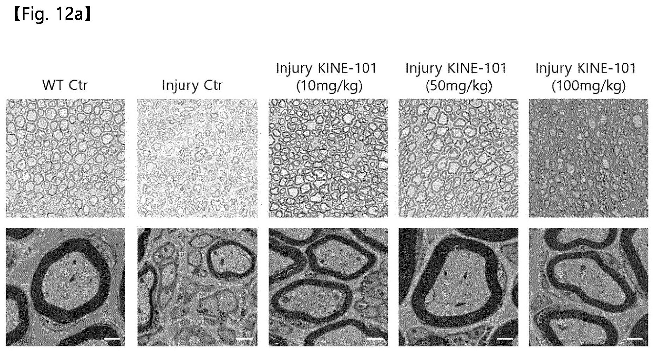

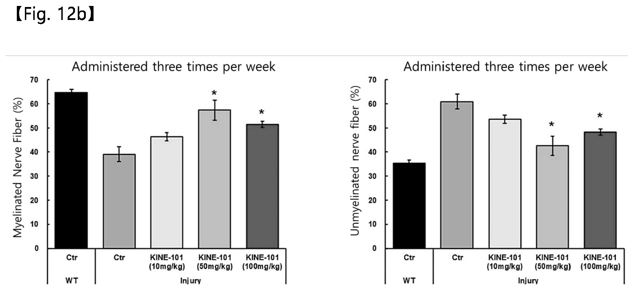

As a result, as shown in Figs. 12a and 12b, the KINE-101-treated groups exhibited improved myelination compared with the acquired peripheral nerve injury group (Injury Ctr). In addition, an increase in the number of large myelinated fibers and a decrease in the number of small unmyelinated fibers were observed. In particular, compared with the acquired peripheral nerve injury group, the KINE-101 50 mg/kg treatment group and the KINE-101 100 mg/kg treatment group showed a marked increase in myelinated axons and a decrease in unmyelinated fibers.

### <4-5> Evaluation of Treg Cell Proportion and Activation in an Acquired Peripheral Nerve Injury Animal Model

After administration of KINE-101 to the acquired peripheral nerve injury animal model, the proportion of Treg cells and the activation of Treg cells present in the lymph nodes at the nerve injury site were evaluated.

Specifically, KINE-101 was administered to the acquired peripheral nerve injury animal model for four weeks according to the method described in Example <4-1>, and the experimental animals were sacrificed six weeks after the start of administration. Thereafter, immune cells were isolated from the lymph nodes at the nerve injury site of the experimental animals. The isolated immune cells were subjected to fluorescent staining for CD4 and Foxp3 proteins, and the proportion of Treg cells among CD4 T cells was determined by flow cytometry. In addition, fluorescent staining for the CTLA4 protein, which is an activation marker of Treg cells, was performed, and the level of CTLA4 expression in Treg cells was compared by flow cytometric analysis to evaluate the degree of activation.

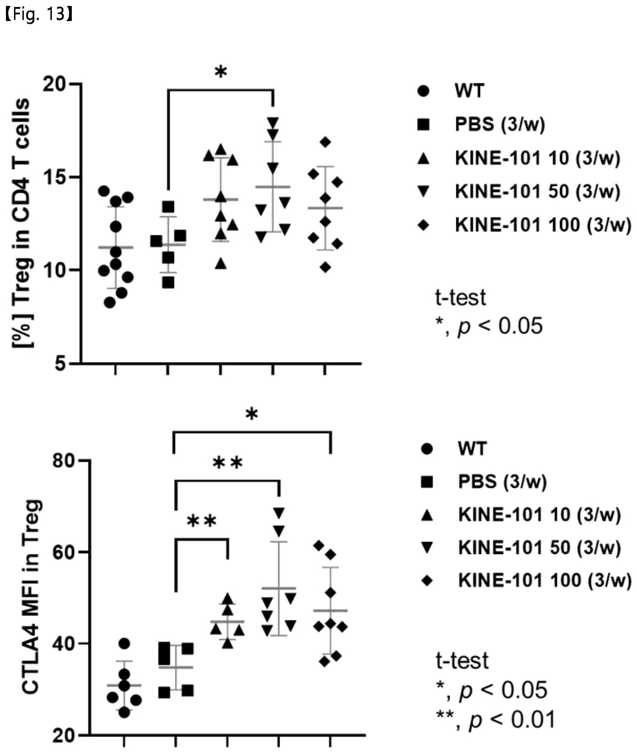

As a result, as shown in Fig. 13, when the proportion of Treg cells present in the lymph nodes at the peripheral nerve injury site in the acquired peripheral nerve injury model was compared with that of the normal group, it was confirmed that the proportion of Treg cells increased in the KINE-101-treated groups. In particular, the proportion of Treg cells in the KINE-101 50 mg/kg treatment group increased to a statistically significant level.

In addition, when the level of CTLA4 expression, which is one of the activation markers of Treg cells, was compared, it was confirmed that CTLA4 expression increased in the KINE-101-treated groups. In particular, CTLA4 expression in the KINE-101 50 mg/kg treatment group increased to a statistically significant level, confirming that activation of Treg cells was induced by administration of KINE-101.

Accordingly, based on the results of Examples <2> to <4>, it was confirmed that the peptide of the present invention suppresses demyelination and enhances myelination in a CIDP animal model and an acquired peripheral nerve injury animal model, and improves nerve damage and symptoms caused by demyelination. Therefore, the peptide of the present invention can be used for the prevention or treatment of demyelinating diseases, including CIDP.

### [Industrial Applicability]

The trimeric peptide according to the present invention suppresses demyelination and enhances myelination in a chronic inflammatory demyelinating polyneuropathy (CIDP) animal model and an acquired peripheral nerve injury animal model, and exhibits effects of improving nerve damage and symptoms caused by demyelination. Accordingly, the peptide can be usefully employed as an active ingredient in a composition for the prevention or treatment of demyelinating diseases, including CIDP.

## Claims

1. A pharmaceutical composition for preventing or treating a demyelinating disease, comprising, as an active ingredient, a peptide consisting of the amino acid sequence set forth in SEQ ID NO: 1 or a polynucleotide encoding the same.

2. The pharmaceutical composition for preventing or treating a demyelinating disease according to claim 1, wherein the N- or C-terminus of the peptide is conjugated with a protecting group selected from the group consisting of an acetyl group, a fluorenylmethoxycarbonyl group, a formyl group, a palmitoyl group, a myristyl group, a stearyl group, and polyethylene glycol (PEG).

3. The pharmaceutical composition for preventing or treating a demyelinating disease according to claim 1, wherein the demyelinating diseases are selected from the group consisting of Charcot-Marie-Tooth disease (CMT), chronic inflammatory demyelinating polyneuropathy (CIDP), idiopathic inflammatory demyelinating disease, hereditary neuropathy, anti-MAG peripheral neuropathy, progressive inflammatory neuropathy, optic neuropathy, Devic's disease, central pontine myelinolysis (CPM), extrapontine myelinolysis (EPM), tabes dorsalis, leukoencephalopathies, demyelinating leukodystrophy, neuromyelitis optica, demyelinating optic neuritis, acute disseminated demyelination, periaxial encephalitis, central demyelination of the corpus callosum, acute transverse myelitis, subacute necrotizing myelitis, and concentric sclerosis.

4. The pharmaceutical composition for preventing or treating a demyelinating disease according to claim 1, wherein the peptide improves demyelination.

5. The pharmaceutical composition for preventing or treating a demyelinating disease according to claim 1, wherein the peptide improves damage to motor nerves and sensory nerves.

6. The pharmaceutical composition for preventing or treating a demyelinating disease according to claim 1, wherein the peptide improves decreases in motor nerve conduction and sensory nerve conduction.

7. The pharmaceutical composition for preventing or treating a demyelinating disease according to claim 1, wherein the composition increases the proportion of Treg cells in lymph nodes at a peripheral nerve injury site and induces activation of the Treg cells.

8. The pharmaceutical composition for preventing or treating a demyelinating disease according to claim 1, wherein the composition is formulated for oral administration, intramuscular administration, intravenous administration, intraperitoneal administration, subcutaneous administration, intradermal administration, or topical administration.

9. A health functional food composition for preventing or ameliorating a demyelinating disease, comprising, as an active ingredient, a peptide consisting of the amino acid sequence set forth in SEQ ID NO: 1 or a polynucleotide encoding the same.

10. A method for treating a demyelinating disease, comprising administering to a subject a therapeutically effective amount of a peptide consisting of the amino acid sequence set forth in SEQ ID NO: 1 or a polynucleotide encoding the same.

11. A method for preventing or ameliorating a demyelinating disease, comprising administering to a subject a peptide consisting of the amino acid sequence set forth in SEQ ID NO: 1 or a polynucleotide encoding the same.

12. Use of a peptide consisting of the amino acid sequence set forth in SEQ ID NO: 1 or a polynucleotide encoding the same for use in a pharmaceutical composition for preventing or treating a demyelinating disease.

13. Use of a peptide consisting of the amino acid sequence set forth in SEQ ID NO: 1 or a polynucleotide encoding the same for use in a health functional food composition for preventing or ameliorating a demyelinating disease.

14. Use of a peptide consisting of the amino acid sequence set forth in SEQ ID NO: 1 or a polynucleotide encoding the same for preparing a pharmaceutical composition for preventing or treating a demyelinating disease.

15. Use of a peptide consisting of the amino acid sequence set forth in SEQ ID NO: 1 or a polynucleotide encoding the same for preparing a health functional food composition for preventing or ameliorating a demyelinating disease.
